**Europäisches Patentamt**

**European Patent Office**

**Office européen des brevets**

(19)

(11) Veröffentlichungsnummer: **0 237 037 B1**

(12) **EUROPÄISCHE PATENTSCHRIFT**

(45) Veröffentlichungstag der Patentschrift: **14.08.91**

(51) Int. Cl.⁵: **A61F 2/06, A61L 27/00**

(21) Anmeldenummer: **87103451.8**

(22) Anmeldetag: **10.03.87**

(54) **Mit vernetzter Gelatine imprägnierte Gefässprothese und Verfahren zu ihrer Herstellung.**

(30) Priorität: **12.03.86 DE 3608158**

(43) Veröffentlichungstag der Anmeldung:
**16.09.87 Patentblatt 87/38**

(45) Bekanntmachung des Hinweises auf die
Patenterteilung:
**14.08.91 Patentblatt 91/33**

(84) Benannte Vertragsstaaten:
**AT BE CH DE ES FR GB GR IT LI LU NL SE**

(56) Entgegenhaltungen:
**DE-A- 1 491 218**
**DE-A- 1 494 939**
**DE-A- 2 734 503**

(73) Patentinhaber: **B. Braun-SSC AG**
**Gerliswilstrasse 74**
**CH-6020 Emmenbrücke(CH)**

(72) Erfinder: **Fleckenstein, Peter, Dr.**
**Am Bürgerhaus 4**
**W-3501 Fuldabrück 1(DE)**
Erfinder: **Werner, Heinz Helmut, Dr. med.**
**Am Steig 2**
**W-3508 Melsungen(DE)**

(74) Vertreter: **Patentanwälte RUFF, BEIER und**
**SCHÖNDORF**
**Neckarstrasse 50**
**W-7000 Stuttgart 1(DE)**

**Beschreibung**

Die Erfindung betrifft eine durch Imprägnierung mit vernetzter Gelatine abgedichtete an sich poröse Gefäßprothese und ein Verfahren zu ihrer Herstellung.

Gefäßprothesen zum Ersatz von Hohlorganen bei Mensch und Tier, insbesondere von Blutgefäßen, sind seit langem bekannt. Sie bestehen in der Regel aus textilem Material, insbesondere einem Gewirk (DE-A2-26 l3 575, DE-A2-20 09 349) oder Gestrick (DE-Al-24 6l 370). Sie können jedoch auch aus nichttextilen Materialien bestehen (vgl. EP-Al-0 l06 496 und GB-Al-l5 06 432). In aller Regel sind die Gefäßprothesen porös, um ein Einwachsen des Gewebes zur Erzielung möglichst natürlicher Verhältnisse zu ermöglichen. Da diese Poren aber nach der Implantation der Prothese häufig zu unerwünscht hohen Verlusten an Körperflüssigkeiten führen können, ist man bestrebt, die Poren mit einem vom Körper resorbierbaren Material abzudichten, das sukzessive durch das einwachsende Gewebe ersetzt wird.

Aus der DE-A2-l4 94 939 ist es bekannt, als Imprägnierungsmittel Prokollagen zu verwenden, das in saurer Lösung auf die poröse Prothese aufgetragen und anschließend durch Erhöhen des pH-Wertes unlöslich gemacht wird.

Die Verwendung von Kollagen zum Abdichten ist bekannt aus der DE-A2-l4 9l 2l8, der US-Al-4l 67 045 sowie der DE-Al-35 03 l27 und der DE-Al-35 03 l26. Die Vernetzung des Kollagens wird normalerweise mit Aldehyden vorgenommen, insbesondere mit Formaldehyd.

Es ist auch schon bekannt, poröse Gefäßprothesen mit löslicher Gelatine zu imprägnieren und diese zu vernetzen (vgl. DE-A2-l4 94 939), wobei die Vernetzung mit Hilfe von Thiolgruppenhaltigen Verbindungen mit nachfolgender oxydativer Vernetzung unter Bildung von Disulfid-Brücken vorgenommen wurde.

An die Eigenschaften der Imprägnierung werden hohe Anforderungen gestellt. Die Imprägnierung soll am Prothesenkörper gut haften, diesen gut abdichten, elastisch sein und, insbesondere bei der Resorption, keine schädlichen Produkte freisetzen. Die bekannten imprägnierten Prothesen erfüllen diese Forderungen nur teilweise. Außerdem ist ihre Herstellung sehr umständlich, oder es sind teuere Ausgangsmaterialien erforderlich, die einer industriellen Fertigung entgegenstehen.

Der Erfindung liegt daher die Aufgabe zugrunde, eine imprägnierte Gefäßprothese zu schaffen, die einfach herstellbar ist, gute mechanische Eigenschaften besitzt und völlig dicht ist, für den Empfängerorganismus unschädlich ist und sich insbesondere bei der Implantation leicht handhaben läßt.

Diese Aufgabe wird gelöst durch eine durch Imprägnierung mit vernetzter Gelatine abgedichtete, an sich poröse Gefäßprothese, die dadurch gekennzeichnet ist, daß die Gelatine mit einem Diisocyanat vernetzt ist.

Die Isocyanate reagieren mit den reaktionsfähigen nukleophilen Gruppen der Gelatine, insbesondere den Amino-Gruppen und auch Hydroxyl-Gruppen unter Bildung stabiler Verknüpfungen und Vernetzungsprodukte. Im Gegensatz zu der Vernetzung mit Aldehyden, bei der sich ein Gleichgewichtszustand einstellt, so daß Aldehyde rückgebildet und wieder freigesetzt werden, ist die Vernetzung mit Diisocyanaten irreversibel. Nach beendeter Reaktion liegen keine Isocyanatreste mehr vor und werden auch nicht aus dem Vernetzungsprodukt rückgebildet. Eine mit Diisocyanat vernetzte Gelatine bildet eine dichte Imprägnierung mit guten mechanischen Eigenschaften, so daß im Verhältnis zur Porosität der Gefäßprothese nur wenig Gelatine und Vernetzungsmittel erforderlich sind und trotzdem eine vollständige Abdichtung erzielt wird.

Die Vernetzung von biologischem Material mit Diisocyanaten ist an sich bekannt. So ist in der DE-A2-27 34 503 ein Verfahren zur Herstellung eines Kollagenschwa mmes beschrieben, bei dem ein Brei aus teilweise abgebautem Kollagen mit Diisocyanat versetzt, auf Temperaturen von -l0 bis -30 °C schockgefroren, bei Temperaturen unter 0 °C belassen, gewaschen und nachbehandelt und dann getrocknet wird. Dabei wird ein weicher, poröser Schwamm erhalten. Bei einem aus der DE-A2-30 20 6ll bekannten Verfahren werden Achillessehnen aufgeschlossen und dann zerfasert, wonach die Fasern unter Verwendung von in einer wäßrigen Salzlösung emulgiertem Hexamethylendiisocyanat vernetzt werden und dann aus den vernetzten Fasern unter anderem verstrickbare Garne hergestellt werden. Diese Verfahren stehen jedoch in keinem Zusammenhang mit der der Erfindung zugrundeliegenden Aufgabe.

Die an sich poröse Gefäßprothese kann eine Porosität im üblichen Rahmen von ca. 2000 cm³/min/cm² haben. Mit Hilfe der erfindungsgemäßen Imprägnierung aus mit Diisocyanaten versetzter Gelatine kann diese Porosität vollständig beseitigt werden. Der Abbau der so vernetzten Gelatine im Körper erfolgt gebremst in dem Maße, in dem das neue Gewebe in den porösen Körper der Gefäßprothese einwächst und für die natürliche Abdichtung sorgt.

Der an sich poröse Körper der Gefäßprothese kann den üblichen Aufbau einer textilen Gefäßprothese haben, so zum Beispiel den eines glatten Kettengewirkes nach der deutschen Patentschrift 20 09 349, den Aufbau einer einseitigen Velourprothese nach der US-Patentschrift 38 78 565 oder den einer Doppelvelourprothese nach der deutschen Patentschrift 26 l3 575. Die Prothese kann aber auch eine poröse, nicht-textile Prothese sein, beispielsweise aus gestrecktem Polytetrafluoräthylen, wie sie im britischen Patent l5 06 432 beschrieben ist. Die die Poren umgrenzenden Strukturen der Prothese sind zur Abdichtung mit einem dünnen Film der vernetzten Gelatine überzogen, wobei die Poren selbst durch dünne Gelatinemembrane verschlossen sind. Durch die filmartige Beschichtung der einzelnen Strukturelemente der Gefäßporothese und die Verbindung der Strukturelemente durch Membranen wird eine Abdichtung erhalten, die sich von einer die Porosität vollständig ausfüllenden bzw. überdeckenden Beschichtung unterscheidet. Beispielsweise sind bei textilen Prothesen Einzelfasern oder Faserbündel mit einem Film vernetzter Gelatine überzogen und durch die Membranen über die zwischen den Fasern bzw. Faserbündeln vorhandenen Zwischen räume hinweg miteinander verbunden und zwar in verschiedenen Ebenen innerhalb der Wandung der Gefäßprothese. Aufgrund dieser Feinstruktur wird die Gefäßprothese durch die Imprägnierung in ihrer Handhabung nicht beeinträchtigt, sondern eher noch verbessert.

Die gute Abdichtung der Gefäßprothese bei Verwendung von nur wenig Imprägnierungsmaterial wird auch dadurch ermöglicht, daß es sich bei Gelatine um ein in homogener Lösung vorliegendes Material handelt, im Gegensatz zu Kollagen, das ein heterogenes Fasermaterial ist und deshalb in dünnen Schichten nicht völlig dicht ist. Das Gewichtsverhältnis von porösem Prothesenkörper zu Imprägnierung kann im Bereich von l:0,2 bis l:3, insbesondere bei ca. l:l liegen, einschließlich der in der Imprägnierung ggf. vorhandenen weiteren Zusätze wie Weichmacher. Hydrophile Weichmacher, insbesondere Glycerin und andere bekannte Polyole werden mit Vorteil verwendet, um ein vollständiges Austrocknen der Imprägnierung zu verhindern und die Elastizität der Imprägnierung zu erhöhen. Der Feuchtigkeitsgehalt der Imprägnierung im getrockneten Zustand liegt vorzugsweise im Bereich von l5 bis 25, insbesondere l7 bis 22 Gew.-%, bezogen auf das Gewicht der vernetzten Imprägnierung.

Hexamethylendiisocyanat ist als Vernetzungsmittel bevorzugt. Es können jedoch auch andere Diisocyanate verwendet werden, insbesondere aliphatische Diisocyanate mit 4 bis l2, vorzugsweise 6 bis l0 C-Atomen. Im Gegensatz zu Formaldehyd hat sich gezeigt, daß ein Vernetzungsmittel größerer Kettenlänge aufgrund der sogenannten Spacer-Funktion der Verbrückungen zwischen den Proteinketten günstiger für die Elastizität des Produkt es ist.

Die erfindungsgemäße Gefäßprothese kann in ihrer Imprägnierung in an sich bekannter Weise therapeutisch wirksame Materialien oder andere Wirkstoffe enthalten. Bei einer besonders bevorzugten Ausführungsform sind diese Materialien bzw. Wirkstoffe jedoch nicht einfach in die Imprägnierung eingemischt, sondern an Träger gebunden bzw. in diese eingeschlossen, so daß sie nur retardiert freigesetzt werden. Zu diesem Zweck kann die Imprägnierung, insbesondere die Gelatine Bestandteile enthalten, die die Wirkstoffe absorbieren bzw. adsorbieren und nur allmählich freigeben. Besonders geeignete Bestandteile dieser Art sind Austauscher, insbesondere Ionenaustauscher. Bei einer besonders bevorzugten Ausführungsform der Erfindung besteht mindestens ein Teil der Gelatine aus succinylierter Gelatine, die derartige Eigenschaften besitzt. Aminoglykoside, wie Gentamycin, können aufgrund ihrer basischen Funktionen durch derartige modifizierte Gelatine gut festgehalten werden. Da die modifizierte Gelatine weniger gut geliert als normale Gelatine, hängt der Anteil an modifizierter Gelatine normalerweise von der Gelierfähigkeit der Mischung ab und kann im Bereich von l0 bis 50 Gew.-% in Bezug auf die Gesamtmenge an Gelatine liegen. Als Gelatine ist im übrigen gut gelierbare Gelatine, wie Speisegelatine, bevorzugt (Bloom-Wert ll0 bis 300, vorzugsweise 240 bis 280). Bei der Vernetzung mit dem Diisocyanat wird die die Austauschereigenschaften besitzende modifizierte Gelatine mit einvernetzt, das therapeutisch wirksame Material bzw. der Wirkstoff haftet somit an der Gelatinestruktur selbst.

Das erfindungsgemäße Verfahren zur Herstellung der imprägnierten Gefäßprothese ist dadurch gekennzeichnet, daß eine poröse Gefäßprothese mit einer wäßrigen Lösung von Gelatine getränkt wird, man die Gelatine gelieren läßt und dann vorsichtig entwässert, insbesondere in Luft trocknet, wonach man die erhaltene Vorimprägnierung mit dem Diisocyanat vernetzt.

Diisocyanate reagieren nicht nur mit der Gelatine, sondern auch mit anderen polare Gruppen aufweisenden Verbindungen, wie beispielsweise mit Wasser und Alkoholen, unter Bildung von unerwünschten Nebenprodukten, wie unlöslichen Urethan- und Harnstoffderivaten. Obwohl die Imprägnierungsschicht aufgrund ihrer Dichtheit ein Auswaschen von etwaigen, in der Schicht gebildeten Nebenprodukten nicht erlaubt, wie es bei den bekannten, mit Diisocyanat vernetzten porösen Faserpodukten der Fall ist, wurde gefunden, daß sich die Neigung der Diisocyanate zur Bildung von Nebenprodukten nicht schädlich auswirkt.

Zum Tränken der porösen Gefäßprothese mit der Gelatinelösung wird die Gefäßprothese vorzugsweise in die Lösung eingetaucht. Durch Anlegen eines Vakuums kann erreicht werden, daß die Poren vollständig mit der Gelatinelösung gefüllt werden. Die Gelatinekonzentration in der Lösung kann innerhalb von weiten Grenzen variieren und liegt vorzugsweise zwischen 3 und 20, insbesondere zwischen 5 und l5 Gew.-% der Imprägnierungslösung. Wichtig ist jedoch das Vorliegen einer homogenen Lösung. Die Imprägnierung erfolgt bei erhöhter Temperatur, d.h. bei Temperaturen von über 40 °C, um durch Abkühlen die Gelierung herbeiführen zu können. Da Gelatine in wäßriger Lösung bei Temperaturen über 60° bei längerem Stehen schon abgebaut wird, sollte die Temperatur nicht wesentlich darüber liegen. Temperaturbereiche zwischen 45 und 70°, insbesondere 55 und 60 °C sind deshalb bevorzugt. Die Imprägnierungslösung kann noch den Weichmacher enthalten und zwar in Mengen bis zu 60 Gew.-%, insbesondere l0 bis 40 Gew.-%. Der Wassergehalt beträgt normalerweise 30 bis 97 Gew.-%, vorzugsweise 50 bis 80 Gew.-%. Dabei werden die Mengenverhältnisse in Abhängigkeit von der Gelierfähigkeit der Gelatine bzw. des Gelatingemisches so aufeinander abgestimmt, daß die Gelatine bei der Imprägnierungstemperatur gelöst ist und die Imprägnierungslösung fließfähig ist, die Gelatine aber bei Abkühlen auf ca. 20 bis 30 °C geliert.

Während der Abkühlung werden die mit der Gelatinelösung getränkten Prothesen vorzugsweise bewegt, um eine gleichmäßige Schichtdicke der gelierten Gelatine zu erhalten. Hierzu können die Prothesen nach dem Herausnehmen aus dem Tränkebad und nach kurzem Abtropfenlassen um ihre Längsachse gedreht oder in Taumelbewegung versetzt werden.

Wichtig ist, daß die Entwässerung der gelierten Gelatineschicht bzw. ihre Trocknung schonend durchgeführt wird, um eine gute und einheitlich strukturierte Imprägnierung zu erhalten. Hierzu eignet sich eine Lufttrocknung bei 25 bis 35, insbesondere ca. 30 °C. Die relative Feuchtigkeit der Trocknungsluft liegt vorzugsweise zwischen 30 und 50 %, insbesondere bei ca. 40 %. Insbesondere wenn die Prothesen in die Gelatinelösung unter Anlegen eines Vakuums getaucht werden, reicht eine einmalige Tränkung bzw. Behandlung zur Erzielung einer völligen Dichtheit aus mit dem Vorteil der damit verbundenen geringen Materialmenge und günstigen mechanischen Eigenschaften.

Zur Vernetzung der Gelatine wird mit einem Überschuß an Diisocyanat gearbeitet und zwar einerseits um eine vollständige Vernetzung der Gelatine zu erzielen und sie damit unlöslich zu machen, und andererseits im Hinblick auf die zu erwartenden Nebenreaktionen. Es wird vorzugsweise ohne Beeinflussung des pH-Wertes gearbeitet. Die Gelatine kann bei ihrem normalen pH-Wert, der normalerweise bei ca. 5,5 liegt, belassen werden. Die Vernetzungsreaktion kann in einem pH-Bereich von 3,5 bis 7,5 durchgeführt werden. Annehmbare Reaktionsdauern von ca. 5 bis l0 Stunden sind bei der Vernetzung bei Raumtemperaturen erzielbar, ohne daß sich die Nebenreaktionen nachteilig auswirken oder besondere Maßnahmen zu treffen sind.

Besonders vorteilhaft für die Durchführung der Vernetzungsreaktion ist die Verwendung von Lösungen des Diisocyanats in polaren organischen Lösungsmitteln, die die vorgetrocknete Gelatineimprägnierung gut benetzen. Hierbei sind Lösungsmittel, die mit Wasser und dem Weichmacher mischbar sind, bevorzugt. Isopropanol eignet sich besonders. Aus der Vernetzungslösung, diffundiert das Diisocyanat in die Gelatineschicht und reagiert mit der Gelatine unter Vernetzung. Die Diffusion des Diisocyanats in die Gelatineschicht erfolgt vorzugsweise sowohl von außen als auch von innen. Hierzu können die Prothesen in die Vernetzungslösung mit Vorteil getaucht werden. Obwohl mit einem erheblichen Überschuß an Diisocyanat gearbeitet wird, der vorzugsweise mehr als dem Zehnfachen der für die Vernetzung erforderlichen stöchiometrischen Menge entspricht, liegt die Konzentration an Diisocyanat in der Vernetzungslösung vorzugsweise unter 3 Gew.-%, um die Nebenreaktionen so weit wie möglich zurückzuhalten, da ja das Diisocyanat auch mit Komponenten der Vernetzungslösung zu reagieren vermag. Die Konzentration des Diisocyanats kann zwischen 0,03 und 3 Gew.-% liegen, vorzugsweise zwischen 0,05 und 0,5 Gew.-%, wobei ca. 0,l Gew.-% bevorzugt sind. Die Vernetzungslösung enthält vorzugsweise auch noch Weichmacher und/oder Wasser und zwar zu dem Zweck, um Weichmacher und Wasser in die Gelatineschicht einzubringen oder eine Auswaschung von Wasser und/oder Weichmacher aus der Gelatineschicht während der Vernetzungsreaktion zu verhindern. Die Konzentration an Weichmacher, insbesondere Glycerin, kann bei 0 bis 60, insbesondere l0 bis 20 Gew.-% liegen. Der Wassergehalt kann in Extremfällen bis zu 70 Gew.-% betragen, liegt jedoch normalerweise wesentlich tiefer, nämlich zwischen 3 und 30 Gew.-%, insbesondere zwischen 5 und l0 Gew.-%. Wesentlich für die bevorzugte Ausführungsform ist, daß der Wassergehalt so niedrig ist, daß das Diisocyanat in der Vernetzungslösung in gelöstem Zustand vorliegt. Durch eine relative Bewegung zwischen Vernetzungslösung und Gefäßprothese kann erreicht werden, daß die Konzentration des Diisocyanats auf der Oberfläche der Prothese möglichst hoch gehalten wird. Bei einer bevorzugten Ausführungsform wird die Vernetzungslösung ständig umgewälzt bzw. umgepumpt, wobei sie gleichzeitig filtriert wird. Hierbei werden unlösliche Nebenprodukte ständig entfernt, so daß Ablagerungen auf der Gefäßprothese und damit Verunreinigungen vermieden werden. Die geringe Konzentration an Diisocyanat erlaubt ein gefahrloses Handhaben der Vernetzungslösung. Die Konzentration an Diisocyanat nimmt während der Vernetzungsreaktion ständig ab, so daß nach beendigter Reaktion eine gefahrlose Entsorgung möglich ist. Es sind jedoch Verfahrensvarianten möglich, indem beispielsweise mit im wesentlichen konstanten Konzentrationen an Diisocyanat gearbeitet wird.

Bei einer besonders bevorzugten Ausführungsform der Erfindung werden der Feuchtigkeitgehalt und vorzugsweise auch der Weichmachergehalt der entwässerten, noch unvernetzten, aber vorgetrockneten Gelatineimprägnierung sowie der Wassergehalt und vorzugsweise auch Weichmachergehalt der Vernetzungslösung so eingestellt, daß sie beim Eintauchen der vorgetrockneten imprägnierten Gefäßprothese in die Vernetzungslösung im wesentlichen miteinander im Gleichgewicht stehen. Dadurch können unerwünschte Konzentrationsänderungen, die beispielsweise ein Auslaugen oder Aufquellen der noch nicht vernetzten Gelatineimprägnierung zur Folge haben könnten, vermieden werden.

Nach Beendigung der Vernetzung ist die Gelatineimprägnierung wasserfest, so daß sie mit Wasser, dem ggf. noch Weichmacher zugesetzt ist, gewaschen und dann schonend getrocknet werden kann. Eine Sterilisierung erfolgt vorzugsweise durch an sich bekannte Bestrahlung.

Weitere Merkmale und Einzelheiten der Erfindung ergeben sich aus der nachfolgenden Beschreibung von bevorzugten Ausführungsformen in Verbindung mit den Unteransprüchen. Hierbei können die jeweiligen Merkmale für sich allein oder zu mehreren in Verbindung miteinander verwirklicht sein.

Beispiel 1

Gestrickte Doppel-Mikrovelour-Gefäßprothesen aus Polyäthylenterephthalat-Fasern, die mit einer Plissierung versehen sind, werden in ein Gestell eingespannt und in eine wäßrige Imprägnierlösung aus Gelatine eingetaucht. Die Imprägnierlösung enthält 7,5 Gew.-% Gelatine und 15 Gew.-% Glycerin als Weichmacher in entmineralisiertem Wasser und hat eine Temperatur von 60 °C.

Über der Gelatinelösung wird nunmehr ein Vakuum angelegt, um die in den textilen Prothesen eingeschlossene Luft restlos zu entfernen. Nach Aufsteigen der Luftblasen läßt man die Prothesen noch ca. 15 Minuten in der Lösung unter reduziertem Druck stehen und bringt den Druck danach wieder auf Normaldruck. Man nimmt die Prothesen dann aus der Imprägnierungslösung heraus und läßt sie kurz abtropfen. Danach werden die Prothesen unter leichter Taumelbewegung auf Normaltemperatur abgekühlt. Nachdem die Beschichtungsmasse geliert ist, werden die beschichteten Prothesen in eine Klimakammer gebracht und darin in Luft mit einer relativen Luftfeuchte von 40 % bei 30 °C getrocknet, bis die Restfeuchte in der Imprägnierung bei ca. 20 % liegt.

Danach werden die vorgetrockneten Prothesen in eine Vernetzungslösung getaucht, die 0,1 Gew.-% Hexamethylendiisocyanat, 15 Gew.-% Glycerin, 8,5 Gew.-% Wasser und 76,4 Gew.-% Isopropanol enthält. In dieser Lösung werden die beschichteten Prothesen 8 Stunden bei Raumtemperatur belassen, während die Lösung gleichzeitig ständig umgepumpt und filtriert wird. Nach Beendigung der Vernetzung werden die Prothesen aus der Vernetzungslösung herausgenommen und mit einer 15 %-igen Lösung von Glycerin in Wasser gewaschen und dann wiederum bei 30 °C und 40 % relative Luftfeuchte schonend getrocknet, bis eine Restfeuchte von 15 bis 20 %, bezogen auf die Imprägnierung, erhalten ist. Die Prothesen werden dann auf Nennlänge zugeschnitten, einzeln verpackt und durch Bestrahlung sterilisiert.

Die auf diese Weise imprägnierten Prothesen sind völlig dicht. Die Imprägnierung sitzt in der textilen Faserstruktur und ist mit dem bloßen Auge kaum zu erkennen. Die Prothese hat ein helles weißes Aussehen. Sie ist flexibel und in axialer Richtung komprimierbar und streckbar. Nach Implantation der Prothese wird das vernetzte Beschichtungsmaterial in dem Maße resorbiert, in dem das natürliche Gewebe nachwächst, ohne daß die Abbauprodukte irgendwelche schädliche Wirkungen erkennen lassen. Auch die Imprägnierung selbst ist sowohl immunologisch als auch toxikologisch unbedenklich.

Beispiel 2

Die Verfahrensweise nach Beispiel 1 wird wiederholt, wobei anstelle von Speisegelatine eine Mischung aus 70 Gew.-% Speisegelatine und 30 Gew.-% succinylierter Gelatine verwendet wird. Die auf diese Weise erhaltene vernetzte Imprägnierung enthält zusätzliche saure Reste, die die Imprägnierung zur Einlagerung, beispielsweise durch Diffusion, und Bindung von therapeutischen Wirkstoffen mit basischen Eigenschaften geeignet machen. Diese therapeutischen Wirkstoffe werden nach der Implantation der Prothese nur sehr langsam freigegeben, so daß beispielsweise ein Infektionsschutz über eine verlängerte Zeitdauer gegeben ist.

Beispiel 3

Gestickte Gefäßprothesen vom Doppelvelourtyp, die aus Polyäthylenterephthalat-Fasern aufgebaut und mit einer Plissierung versehen sind, werden wie in Beispiel 1 in ein Gestell eingespannt und dann im Tauchverfahren mit Gelatine imprägniert. Die wäßrige Imprägnierlösung enthält 10 Gew.-% Gelatine und 20 Gew.-% Glycerin und hat eine Temperatur von 60 °C. Wie in Beispiel 1 werden die Textilprothesen bei reduziertem Druck zunächst gründlich entgast. Nach Entnahme läßt man dann kurz abtropfen und hält das Gestell während der Gelierphase der Imprägnierlösung in leichter Taumelbewegung. Nach Verfestigung der Beschichtungsmasse werden die imprägnierten Prothesen in eine Klimakammer transferiert und wie bei Beispiel I einer schonenden Zwischentrocknung unterworfen, bis die Restfeuchte in der Imprägnierung bei etwa 25 % liegt. Danach werden die vorgetrockneten Prothesen im aufgespannten Zustand in ein Vernetzerbad gegeben, das 0,2 Gew.-% Hexamethylendiisocyanat, 50 Gew.-% Glycerin, 43,3 Gew.-% Isopropanol und 6,5 Gew.-% Wasser enthält. Wie in Beispiel I werden die beschichteten Prothesen 8 Stunden bei Raumtemperatur in der Vernetzerlösung belassen. Der anschließende Spülschritt erfolgt in der Weise, daß die aufgespannten Prothesen ca. 5 Minuten einer 30 %-igen wäßrigen Glycerinlösung bei Raumtemperatur im Umlaufverfahren ausgesetzt werden.

Nach Trocknung weist die Imprägnierung der so hergestellten Prothesen eine Restfeuchte von etwa 22 % auf.

Die beschichtete Prothese nach Beispiel 1 enthält

```
        9,5 Gew.-% Wasser

     22    Gew.-% Glycerin

     18    Gew.-% vernetzte Gelatine
     ─────────────────────────────

d.h.  49,5 Gew.-% Imprägnierung  und

      50,5 Gew.-% porösen Grundkörper.
```

Die Prothese nach Beispiel 3 enthält

```
     13   Gew.-% Wasser

     32   Gew.-% Glycerin

     16   Gew.-% vernetzte Gelatine
     ─────────────────────────────

d.h. 61   Gew.-% Imprägnierung  und

     39   Gew.-% gestrickte poröse Prothese.
```

Im allgemeinen kann die Imprägnierung, bezogen auf das Gesamtgewicht der Imprägnierung,

```
10 - 30 Gew.-% Wasser

10 - 60 Gew.-% Weichmacher  und

20 - 60 Gew.-% vernetzte Gelatine
```

enthalten.

**Patentansprüche**

1. Durch Imprägnierung mit vernetzter Gelatine abgedichtete an sich poröse Gefäßprothese, dadurch gekennzeichnet, daß die Gelatine mit einem Diisocyanat vernetzt ist.

2. Gefäßprothese nach Anspruch I, dadurch gekennzeichnet, daß die an sich poröse Gefäßprothese eine textile, insbesondere eine gewirkte Gefäßprothese ist.

3. Gefäßprothese nach Anspruch I oder 2, dadurch gekennzeichnet, daß die die Poren umgrenzenden Strukturen der Gefäßprothese mit einem dünnen Film von vernetzter Gelatine überzogen und die Poren durch Gelatinemembranen verschlossen sind.

4. Prothese nach einem der Ansprüche I bis 3, dadurch gekennzeichnet, daß das Gewichtsverhältnis von an sich poröser Gefäßprothese zur Imprägnierung bei I:0,2 bis I:3, vorzugsweise bei ca. I:I liegt.

5. Gefäßprothese nach einem der vorhergehenden Ansprüche, dadurch gekennzeichnet, daß die Imprägnierung einen hydrophilen Weichmacher, insbesondere Glycerin enthält.

6. Gefäßprothese nach einem der vorhergehenden Ansprüche, dadurch gekennzeichnet, daß die Imprägnierung I0 bis 30 Gew.-%, insbesondere I7 bis 22 Gew.-% Wasser, bezogen auf das Gewicht der Imprägnierung, enthält.

7. Gefäßprothese nach einem der vorhergehenden Ansprüche, dadurch gekennzeichnet, daß das Diisocyanat ein aliphatisches Diisocyanat, insbesondere Hexamethylendiisocyanat ist.

8. Gefäßprothese nach einem der vorhergehenden Ansprüche, dadurch gekennzeichnet, daß in die Imprägnierung mindestens ein therapeutisch wirksames Material eingelagert ist, das vorzugsweise zur Erzeugung eines Retard-Effektes depotartig eingelagert ist und hierzu die Imprägnierung, insbesondere die Gelatine vorzugsweise Bestandteile mit Austauschereigenschaften, insbesondere Ionenaustauschereigenschaften, enthält.

9. Verfahren zur Herstellung der imprägnierten Gefäßprothese nach einem der vorhergehenden Ansprüche, dadurch gekennzeichnet, daß man eine poröse Gefäßprothese mit einer wäßrigen Lösung von Gelatine tränkt, die Gelatine gelieren läßt, die Gelatine danach schonend partiell entwässert, insbesondere in Luft trocknet, und dann die erhaltene Imprägnierung mit dem Diisocyanat vernetzt.

10. Verfahren nach Anspruch 9, dadurch gekennzeichnet, daß das Tränken der porösen Gefäßprothese mit der Gelatinelösung durch Tauchen, insbesondere unter Vakuum, vorgenommen wird.

11. Verfahren nach Anspruch 9 oder I0, dadurch gekennzeichnet, daß die poröse Gefäßprothese nur einmal mit der Gelatinelösung behandelt wird.

12. Verfahren nach einem der Ansprüche 9 bis II, dadurch gekennzeichnet, daß zur Vernetzung der Gelatineimprägnierung eine Lösung des Diisocyanats in einem organischen Lösungsmittel verwendet wird, das vorzugsweise mit Wasser und ggf. dem Weichmacher mischbar ist, insbesondere zur Vernetzung der Gelatineimprägnierung eine Lösung aus

```
     0,03 bis   3 Gew.-% Diisocyanat, insbesondere
                         Hexamethylendiisocyanat,
        0    bis 60 Gew.-% Weichmacher, insbesondere
                         Glycerin,
       30    bis 95 Gew.-% Lösungsmittel, insbesondere
                         Isopropanol und
        5    bis 70 Gew.-% Wasser
```

verwendet wird, wobei die Mengenverhältnisse so aufeinander abgestimmt sind, daß das Diisocyanat in gelöstem Zustand vorliegt.

**13.** Verfahren nach einem der Ansprüche 9 bis l2, dadurch gekennzeichnet, daß die mit der Gelatine imprägnierte Gefäßprothese in die Vernetzungslösung getaucht und relativ zu dieser bewegt wird, wobei die Vernetzungslösung vorzugsweise während der Vernetzungsreaktion ständig umgewälzt und dabei durch Filtration von unlöslichen Nebenprodukten befreit wird.

**14.** Verfahren nach einem der Ansprüche 9 bis l3, dadurch gekennzeichnet, daß der Feuchtigkeitsgehalt und vorzugsweise auch der Weichmachergehalt der partiell entwässerten, noch nicht vernetzten Gelatineimprägnierung so eingestellt wird, daß er im wesentlichen im Gleichgewicht mit dem Wassergehalt und ggf. Weichmachergehalt in der Vernetzungslösung steht.

## Claims

**1.** Vessel prosthesis which is porous as such which is sealed by impregnating with crosslinked gelatin, characterized in that the gelatin is crosslinked with a diisocyanate.

**2.** Vessel prosthesis according to claim 1, characterized in that the vessel prosthesis which is porous as such is a textile, particularly a knitted vessel prosthesis.

**3.** Vessel prosthesis according to claim 1 or 2, characterized in that the structures of the vessel prosthesis bounding the pores are coated with a thin film of crosslinked gelatin and the pores are closed by gelatin membranes.

**4.** Vessel prosthesis according to one of the claims 1 to 3, characterized in that the weight ratio of the porous vessel prosthesis to the impregnating coating is 1:0.2 to 1:3, preferably approximately 1:1.

**5.** Vessel prosthesis according to one of the preceding claims, characterized in that the impregnating coating contains a hydrophilic plasticizer, particularly glycerol.

**6.** Vessel prosthesis according to one of the preceding claims, characterized in that the impregnating coating contains 10 to 30% by weight, particularly 17 to 22% by weight of water, used on the weight of the impregnating coating.

**7.** Vessel prosthesis according to one of the preceding claims, characterized in that the diisocyanate is an aliphatic diisocyanate, particularly hexamethylene diisocyanate.

**8.** Vessel prosthesis according to one of the preceding claims, characterized in that in the impregnating coating is incorporated at least one therapeutically active material, which is preferably incorporated in depot-like manner for producing a retard effect and for this purpose the impregnating coating, particularly the gelatin, preferably contains constituents with exchanger characteristics, particularly ion exchanger characteristics.

**9.** Process for producing impregnated vessel prostheses according to one of the preceding claims, characterized in that a porous vessel prosthesis is impregnated with an aqueous gelatin solution, the gelatin is allowed to gel, the gelatin is then carefully partially dehydrated and in particular dried in air and then the impregnating coating obtained is crosslinked with the diisocyanate.

**10.** Process according to claim 9, characterized in that the impregnation of the porous vessel prosthesis with the gelatin solution takes place by immersion, particularly under a vacuum.

**11.** Process according to claim 9 or 10, characterized in that the porous vessel prosthesis is only treated once with the gelatin solution.

**12.** Process according to one of the claims 9 to 11, characterized in that for crosslinking the gelatin impregnating coating use is made of a solution of diisocyanate in an organic solvent, which is preferably miscible with water and optionally the plasticizer and in particular for crosslinking the gelatin impregnating coating use is made of a solution of 0.03 to 3% by weight of diisocyanate, particularly hexamethylene diisocyanate, 0 to 60% by weight of plasticizer, particularly glycerol, 30 to 95% by weight of solvent, particularly isopropanol and 5 to 70% by weight of water, the quantity ratios being so matched to one another that the diisocyanate is present in the dissolved state.

**13.** Process according to one of the claims 9 to 12, characterized in that the gelatin-impregnated vessel prosthesis is immersed in the crosslinking solution and moved relative thereto and preferably the crosslinking solution is constantly circulated during the crosslinking reaction and insoluble by-products are removed by filtration.

**14.** Process according to one of the claims 9 to 13, characterized in that the moisture content and preferably also the plasticizer content of the partly dehydrated, not yet crosslinked gelatin impregnating coating is adjusted in such a way that it is substantially in equilibrium with the water content and optionally the plasticizer content of the crosslinking solution.

**Revendications**

**1.** Prothèse vasculaire poreuse en soi étanchéifiée par imprégnation avec de la gélatine réticulée caractérisée en ce que la gélatine est réticulée avec un diisocyanate.

**2.** Prothèse vasculaire selon la revendication 1 caractérisée en ce que la prothèse vasculaire poreuse en soi est une prothèse vasculaire textile en particulier maillée.

**3.** Prothèse vasculaire selon la revendication 1 ou 2 caractérisée en ce que les structures délimitant les pores de la prothèse vasculaire sont recouverts d'une mince pellicule de gélatine réticulée et que les pores sont fermées par des membranes de gélatine.

**4.** Prothèse selon une des revendications 1 à 3 caractérisée en ce que le rapport pondéral de la prothèse vasculaire poreuse en soi par rapport à l'imprégnation est de 1:0,2 à 1:3, de préférence de 1:1.

**5.** Prothèse vasculaire selon une des revendications précédentes caractérisée en ce que l'imprégnation contient un plastifiant hydrophile, en particulier de la glycérine.

**6.** Prothèse vasculaire selon une des revendications précédentes caractérisée en ce que l'imprégnation contient 10 à 30 % en poids d'eau, en particulier 17 à 22 % en poids d'eau par rapport au poids de l'imprégnation.

**7.** Prothèse vasculaire selon une des revendications précédentes caractérisée en ce que le diisocyanate est un diisocyanate aliphatique, plus particulièrement du diisocyanate d'hexaméthylène.

**8.** Prothèse vasculaire selon une des revendications précédentes caractérisée en ce que l'imprégnation contient au moins une substance à action thérapeutique qui est de préférence intégrée sous forme de dépôt pour la production d'un effet retard et, à cet effet, l'imprégnation, en particulier la gélatine, contient de préférence des éléments à propriétés d'échangeur, plus particulièrement des propriétés d'échangeur d'ions.

**9.** Procédé de fabrication d'une prothèse vasculaire imprégnée selon une des revendications précédentes caractérisé en ce qu'on immerge une prothèse vasculaire poreuse dans une solution aqueuse de gélatine, laisse se gélifier la gélatine, procède ensuite à une déshydratation partielle délicate de la gélatine, en particulier en la séchant à l'air, et réticule ensuite l'imprégnation obtenue avec le diisocyanate.

**10.** Procédé selon la revendication 9 caractérisé en ce que l'imbibition de la prothèse vasculaire poreuse avec la solution de gélatine est assurée par immersion, en particulier sous vide.

**11.** Procédé selon la revendication 9 ou 10 caractérisé en ce que la prothèse vasculaire poreuse n'est traitée qu'une seule fois avec la solution de gélatine.

**12.** Procédé selon une des revendications 9 à 11 caractérisé en ce qu'une solution de diisocyanate dans un solvant organique est utilisée pour la réticulation de l'imprégnation de la gélatine, ladite solution pouvant de préférence être mélangée à de l'eau et éventuellement à un plastifiant, en particulier, pour la réticulation de l'imprégnation de gélatine, une solution de

| | | | | |
|---|---|---|---|---|
| 0,03 à | 3 % | en poids de | diisocyanate, en particulier du diisocyanate d'hexaméthylène, |
| 0 à | 60 % | en poids de | plastifiant, en particulier de glycérine, |
| 30 à | 95 % | en poids de | solvant, en particulier d'isopropanol et |
| 5 à | 70 % | en poids d' | eau |

est utilisée, les rapports quantitatifs étant équilibrés entre eux de telle sorte que le diisocyanate se trouve à l'état dissous.

13. Procédé selon une des revendications 9 à 12 caractérisé en ce que la prothèse vasculaire imprégnée avec la gélatine est immergée dans la solution de réticulation et agitée relativement à celle-ci, la solution de réticulation étant de préférence agitée en permanence pendant la réaction de réticulation et étant ensuite dégagée par filtration des produits secondaires non solubles.

14. Procédé selon une des revendications 9 à 13 caractérisé en ce que le taux d'humidité et de préférence également le taux de plastifiant de l'imprégnation de gélatine partiellement déshydratée mais non encore réticulée sont réglés de telle sorte qu'ils soient principalement en équilibre par rapport à la teneur en eau et éventuellement en plastifiant de la solution de réticulation.